# EUROPÄISCHE PATENTANMELDUNG

(11) **EP 0 591 714 A2**
(43) Veröffentlichungstag der Anmeldung: **13.04.1994**
(21) Anmeldenummer: 93114726.8
(22) Anmeldetag: 14.09.1993
(51) Int. Cl.: A61M 39/00, A61M 5/162, A61M 39/04

(54) **Vorrichtung zum Entnehmen einer Flüssigkeit aus einem medizinischen Behälter**

(30) Priorität: 18.09.1992 DE 9212588 U
(71) Anmelder: Fresenius AG, D-61350 Bad Homburg (DE)
(72) Erfinder: Polzer, Karlheinz, D-65760 Eschborn (DE); Müller, Bernd, D-31226 Peine (DE)
(74) Vertreter: Fuchs, Luderschmidt & Partner Patentanwälte

(57) **Zusammenfassung**

Die Erfindung betrifft eine Vorrichtung zum Entnehmen einer Flüssigkeit (22) aus einem medizinischen Behälter (19) mit einem luft- und flüssigkeitsdichten elastischen Verschluß (23), der einen hintergreifbaren Rand (27) aufweist. Darüber hinaus besitzt die Vorrichtung eine Schlauchleitung (1) mit einem eine Öffnung (9) enthaltenden hohlen Anstechdorn (3) an ihrem vorderen Ende. Der Anstechdorn (3) ist mit einem Griffteil (11) versehen, das zumindest ein wippenartig betätigbares Greifelement (13, 15) enthält, welches den Rand (27) des Verschlusses (23) des Behälters (19) hintergreift, wenn der Anstechdorn (3) den Verschluß (23) durchstoßen hat.

## Beschreibung

Die Erfindung betrifft eine Vorrichtung zum Entnehmen einer Flüssigkeit aus einem medizinischen Behälter mit einem luft- und flüssigkeitsdichten elastischen Verschluß, der einen hintergreifbaren Rand besitzt, wobei die Vorrichtung eine Schlauchleitung mit einem eine Öffnung enthaltenden hohlen Anstechdorn an ihrem vorderen Ende aufweist.

Medizinische Lösungen oder Gemische für die enterale Ernährung, Spülflüssigkeiten und andere Flüssigkeiten werden üblicherweise unter sterilen Bedingungen in Behälter eingefüllt, die dann mit einem luft- und flüssigkeitsdichten elastischen Verschluß, beispielsweise einer elastischen Membran oder einem Stopfen verschlossen werden. Solche medizinischen Behälter können Glasflaschen oder auch Kunststoffbeutel sein. Glasflaschen an sich sind allgemein bekannt, jedoch ist aus DE-U-90 01 159 ein Glasgefäß mit einem durchstechbaren Verschluß bekannt, der einen hintergreifbaren Rand besitzt.

In diesem Gebrauchsmuster ist auch ein Kunststoffbeutel bekannt, der Anschlußstutzen zum Zuspritzen oder Ableiten von Flüssigkeiten aufweist.

Eine Steckkupplung, wie sie beispielsweise an einem Anstechdorn angebracht sein kann, ist aus der US 3,394,958 bekannt. Beim weiblichen Steckelement handelt es sich um ein hülsenartiges Teil, das an seinem vorderen Ende eine ringförmige Rastschulter aufweist, die einen Absatz des männlichen Steckelementes hintergreift. Eine solche Verbindung ist schwer zu kuppeln, da ein großer Kraftaufwand erforderlich ist, um die ringförmige Rastschulter über das männliche Steckelement, das ein Verschluß eines Behälters sein kann, zu zwingen. Ein entsprechender Kraftaufwand ist beim Lösen der Steckkupplung erforderlich.

Aus dem DE-U-89 13 766 und aus dem bereits erwähnten DE-U-90 01 159 sind Anstechdorne bekannt, die im wesentlichen eine kegelige oder pyramidenförmige Spitze aufweisen. Die Anstechdorne sind hohl ausgebildet und besitzen eine Öffnung zum Ableiten der Flüssigkeit ins Innere des Anstechdorns. Solche Anstechdorne sind beispielsweise an Tropfkammern vorgesehen. Darüberhinaus ist aus der US 4,201,406 ein am Ende einer Schlauchleitung angebrachter Anstechdorn bekannt, der in einen mit Membran versehenen Verschluß eines Kunststoffbeutels einführbar ist. Ein Flanschteil ist ebenfalls am Anstechdorn angebracht und überdeckt mit seinem Bund das Ende des Verschlusses des Schlauchbeutels und dient gleichzeitig als Anschlag für dieses Verschlußende. Eine Arretierung des Anstechdornes am Verschluß des Beutels ist nicht vorgesehen, so daß der Anstechdorn bei unbeabsichtigter Manipulation aus dem Verschluß des Beutels herausrutschen kann.

Der Erfindung liegt daher die Aufgabe zugrunde, eine Vorrichtung zum Entnehmen einer Flüssigkeit aus einem medizinischen Behälter zu schaffen, mit der der Anstechdorn der Vorrichtung so am Behälter fixiert werden kann, daß er nicht durch unbeabsichtigte Manipulation aus dem Behälter entgleitet, wobei die Vorrichtung mehrfach wiederverwendbar sein soll.

Erfindungsgemäß wird die Aufgabe durch eine Vorrichtung zum Entnehmen einer Flüssigkeit aus einem medizinischen Behälter mit einem luft- und flüssigkeitsdichten elastischen Verschluß, der einen hintergreifbaren Rand besitzt, gelöst, wobei die Vorrichtung eine Schlauchleitung mit einem eine Öffnung enthaltenden hohlen Anstechdorn an ihrem vorderen Ende versehen ist, der eine Griffteil aufweist, das zumindest ein wippenartig betätigbares Greifelement enthält, welches den Rand des Verschlusses des Behälters hintergreift, wenn der Anstechdorn den Verschluß durchstoßen hat.

In weiterer Ausbildung der Erfindung ist das wippenartig betätigbare Greifelement ein längliches, parallel zum Anstechdorn verlaufendes Teil, das mit seinem mittleren Abschnitt, der als Schwenkpunkt dient, am Schaft des Anstechdorns befestigt ist, und wobei der vordere Abschnitt des länglichen Teils einen radial zur Längsachse des Anstechdorns gebogenen Vorsprung zum Hintergreifen des Verschlußrandes des Behälters aufweist.

In weiterer Ausbildung der Erfindung kann dem wippenartig betätigbaren Greifelement gegenüberliegend ein weiteres wippenartig betätigbares Greifelement gleicher Bauart zugeordnet sein.

Der jeweils hintere Abschnitt des länglichen Teils des Greifelementes ist zur Längsachse des Anstechdorns hin betätigbar.

Um die Flüssigkeit aus einem medizinischen Behälter zu entnehmen, wird die Membran bzw. der Stopfen des Verschlusses des Behälters mit dem Anstechdorn durchstoßen. Damit die neuerungsgemäße Vorrichtung am Behälterverschluß arretiert werden kann, werden die Greifelemente des Griffteils an ihren hinteren Abschnitten wippenartig durch gleichseitigen Druck auf diese Wippflächen zur Längsachse des Anstechdornes hin betätigt, so daß sich die vorderen Abschnitte der Greifelemente des Griffteils öffnen. Nach dem Einführen des Anstechdorns in den Verschluß des Beutels gleitet der Verschluß an den an den vorderen Abschnitten der Greifelemente befindlichen Vorsprüngen vorbei, so daß die Vorsprünge den Rand des Verschlusses an seiner Hinterseite hintergreifen. Wird nun kein Druck mehr auf die hinteren Abschnitte der Greifelemente ausgeübt, nehmen die Greifelemente aufgrund ihrer federelastischen Materialeigenschaften ihre Ausgangsstellung ein, so daß die neuerungsgemäße Vorrichtung am Verschluß des Behälters verriegelt ist. Über die Öffnung des Anstechdornes fließt die Flüssigkeit aus dem medizinischen Behälter in den hohlen Innenraum des Anstechdorns und von dort weiter über die Schlauchleitung zu einem Patienten. Eine Dekonnektierung der Vorrichtung vom Beutel ist durch Druck auf die hinteren Abschnitte der Greifelemente möglich, da sich dadurch die vorderen Abschnitte der Greifelemente auseinander und außer Eingriff mit dem Verschluß des Behälters bewegen. Dadurch kann die Vorrichtung aus dem Verschluß des Behälters entfernt werden und steht für eine zweite Punktion zur Verfügung.

Durch diese neuerungsgemäße Lösung ist es möglich, nach dem Anstechen des Verschlusses des medizinischen Behälters durch den Anstechdorn die Vorrichtung so zu fixieren, daß sie durch unbeabsichtigte Manipulation nicht aus dem Verschluß des Behälters gleiten kann. Ein Wechsel des medizinischen Behälters unter Wiederverwendung der neuerungsgemäßen Vorrichtung ist dadurch gewährleistet.

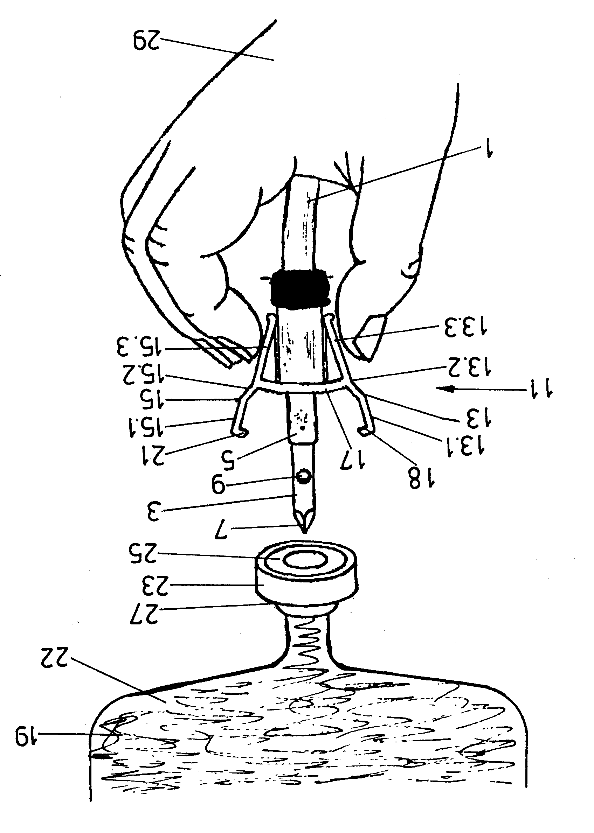

An einem Ausführungsbeispiel wird die Erfindung näher erläutert. Die beigefügte Zeichnung bezieht sich dabei auf eine Vorrichtung, die zwei sich gegenüberliegende Greifelemente aufweist.

Die Vorrichtung zum Entnehmen einer Flüssigkeit aus einem medizinischen Behälter besteht aus einer Schlauchleitung 1, deren nicht dargestelltes Ende beispielsweise mit einer Injektionskanüle, die in den Arm eines Patienten eingeführt sein kann, verbunden ist. Das andere, aus der Zeichnung ersichtliche Ende der Schlauchleitung ist mit einem Anstechdorn 3 versehen, der über eine Schaftteil 5 mit dem Vorderende der Schlauchleitung 1 verbunden ist.

Der Anstechdorn 3 besitzt eine pyramidenförmige Spitze 7 und an seinem Schaft eine Öffnung 9, die in den hohlen Innenraum des Anstechdornes 3 führt und damit eine Verbindung zum Innenraum der Schlauchleitung 1 herstellt.

Unterhalb der Öffnung 9 des Anstechdornes 3 ist ein Griffteil 11 angeordnet. Dieses Griffteil 11 weist zwei parallel zur Längsachse der Schlauchleitung 1 angeordnete und sich gegenüberliegende Greifelemente 13 und 15 auf, die jeweils einen vorderen Abschnitt 13.1, 15.1, jeweils einen mittleren Abschnitt 13.2, 15.2 und jeweils einen hinteren Abschnitt 13.3, 15.3 besitzen. Über einen Steg 17 sind die mittleren Abschnitte 13.2 und 15.2 der Greifelemente 13 und 15 federelastisch verbunden, wobei die mittleren Abschnitte 13.2 und 15.2 der Greifelemente 13 und 15 im wesentlichen als Wipp- bzw. Schwenkpunkt dienen.

Am vorderen Ende der vorderen Abschnitte 13.1 und 15.1 der Greifelemente 13 und 15 sind radial zur Längsachse des Anstechdornes 3 hin gerichtete Vorsprünge 18 und 21 vorgesehen, so daß die vorderen Abschnitte 13.1 und 15.1 der Greifelemente 13 und 15 gewissermaßen eine Zangenform bilden.

Ein medizinischer Behälter 19, der eine Glasflasche oder ein Kunststoffbeutel sein kann, enthält eine medizinische Flüssigkeit 22, beispielsweise eine Spüllösung oder eine Infusionslösung. Der Behälter 19 ist mit einem Verschluß 23 versehen, der eine Membran oder einen Stopfen 25 aus einem elastischen und sich selbst verschließendem Material besitzt. Der Verschluß 23 ist derart ausgebildet, daß dieser einen hintergreifbaren hinteren Rand 27 aufweist.

Um die Vorrichtung zum Zwecke der Entnahme der medizinischen Flüssigkeit 22 in die Schlauchleitung zur Weiterleitung zu einem Patienten mit dem Behälter 19 in Verbindung zu bringen, werden die hinteren Abschnitte 13.3 und 15.3 der Greifelemente 13 und 15 durch die Hand 29 eines Bedieners hin zur Längsachse der Schlauchleitung 1 gedrückt, was geeigneterweise durch Daumen und Zeigefinger geschieht. Dabei werden die Greifelemente 13 und 15 um ihre mittleren Abschnitte 13.2 und 15.2 wippenartig verschwenkt, sodaß sich die vorderen Abschnitte 13.1 und 15.1 der Greifelemente 13 und 15 auseinanderbewegen, so daß der Abstand zwischen den Vorsprüngen 18 und 21 vergrößert wird. Der Anstechdorn 9 der Vorrichtung wird nun durch die Membran bzw. den Stopfen 25 des Verschlusses 23 des Behälters 19 gestoßen, so daß die Spitze 7 des Anstechdornes 3 durch den Verschluß 23 ins Innere des medizinischen Behälters 19 tritt und zwar soweit, daß die Öffnung 9 des Anstechdornes 3 die im Behälter 19 befindliche Flüssigkeit 22 aufnehmen und ins Innere der Schlauchleitung 1 leiten kann.

Wird der Anstechdorn 3 durch den Verschluß 23 des Behälters 19 eingeführt, passieren die Vorsprünge 18 und 21 des Griffteils 11 den Verschluß 23, und zwar soweit, bis sich die Vorsprünge 18 und 21 des Griffteils 11 hinter dem Verschluß 23 befinden. Läßt der Druck der Hand 29 des Bedieners die hinteren Abschnitte 13.3 und 15.3 der Greifelemente 13 und 15 los, werden die Greifelemente durch die federelastische Wirkung ihres Materials in ihre Ausgangsstellung zurückbewegt. Das bedeutet, daß sich die hinteren Abschnitte 13.3 und 15.3 der Greifelemente 13 und 15 wieder radial in bezug auf die Längsachse der Schlauchleitung 1 nach außen bewegen, wodurch die Vorsprünge 18 und 21 der Greifelemente 13 und 15 wieder aufeinander zu bewegt werden. Dadurch hintergreifen sie den Rand 27 des Verschlusses 23, so daß die Vorrichtung fest am medizinischen Behälter 19 arretiert ist.

Nach Beendigung der Entnahme der Flüssigkeit 22 aus dem Behälter 19 wird durch Betätigen der hinteren Abschnitte 13.3 und 15.3 der Greifelemente 13 und 15 der Eingriff des Griffteils vom Rand 27 des Verschlusses 23 gelöst und der Anstechdorn 3 mit der Schlauchleitung 1 aus dem Verschluß 23 des Behälters 19 herausgezogen. Die Vorrichtung kann nun auf gleiche Weise mit einem anderen Behälter verbunden werden.

## Patentansprüche

1. Vorrichtung zum Entnehmen einer Flüssigkeit aus einem medizinischen Behälter (19) mit einem luft- und flüssigkeitsdichten elastischen Verschluß (23), der einen hintergreifbaren Rand (27) besitzt, aufweisend eine Schlauchleitung (1) mit einem eine Öffnung (9) enthaltenden hohlen Anstechdorn (3) an ihrem vorderen Ende, wobei der Anstechdorn (3) mit einem Griffteil (11) versehen ist, das zumindest ein wippenartig betätigbares Greifelement (13, 15) enthält, welches den Rand (27) des Verschlusses (23) des Behälters (19) hintergreift, wenn der Anstechdorn (3) den Verschluß (23) durchstoßen hat.

2. Vorrichtung nach Anspruch 1, dadurch gekennzeichnet, daß das wippenartig betätigbare Greifelement (13, 15) ein längliches, parallel zum Anstechdorn (3) verlaufendes Teil ist, das mit seinem mittleren Abschnitt (13.2, 15.2), der als Schwenkpunkt dient, am Schaft des Anstechdorns (3) befestigt ist, und daß der vordere Abschnitt (13.1, 15.1) des länglichen Teils einen radial zur Längsachse des Anstechdorns (3) gebogenen Vorsprung (18, 21) zum Hintergreifen des Verschlußrandes (27) des Behälters (19) aufweist.

3. Vorrichtung nach den Ansprüchen 1 und 2, dadurch gekennzeichnet, daß dem wippenartig betätigbaren Greifelement (13) gegenüberliegend ein weiteres wippenartig betätigbares Greifelement (15) gemäß Anspruch 2 angeordnet ist.

4. Vorrichtung nach den Ansprüchen 1 bis 3, dadurch gekennzeichnet, daß der jeweils hintere Abschnitt (13.3, 15.3) des länglichen Teils des Greifelementes (13, 15) zur Längsachse des Anstechdorns (3) hin betätigbar ist.

5. Vorrichtung nach den Ansprüchen 1 bis 4, dadurch gekennzeichnet, daß die wippenartig betätigbaren Greifelemente (13, 15) aus einem federelastischen Material, vorzugsweise Kunststoff bestehen.
